# EUROPÄISCHE PATENTSCHRIFT

(11) **EP 0 550 535 B1**
(45) Veröffentlichungstag und Bekanntmachung des Hinweises auf die Patenterteilung: **19.07.1995**
(21) Anmeldenummer: 91916869.0
(22) Anmeldetag: 25.09.1991
(51) Int. Cl.: C07C 205/26, C07C 201/12

(54) **VERFAHREN ZUR HERSTELLUNG VON 2,3-DIFLUOR-6-NITROPHENOL**
PROCESS FOR PRODUCING 2,3-DIFLUOR-6-NITROPHENOL
PROCEDE DE PRODUCTION DE 2,3-DIFLUOR-6-NITROPHENOL

(30) Priorität: 25.09.1990 DE 4030263
(43) Veröffentlichungstag der Anmeldung: 14.07.1993
(73) Patentinhaber: HOECHST AKTIENGESELLSCHAFT, 65926 Frankfurt am Main (DE)
(72) Erfinder: PAPENFUHS, Theodor, D-6000 Frankfurt am Main (DE); HACKENBRUCH, Joachim, D-6500 Mainz (DE); PFIRMANN, Ralf, DR., D-60329 Frankfurt am Main (DE)
(86) Internationale Anmeldenummer: EP9101829
(87) Internationale Veröffentlichungsnummer: WO9205141

(56) Entgegenhaltungen:
- JP-A-62 181 241
- US-A- 4 596 893
- CHEMICAL ABSTRACTS, vol. 107, no. 25, 1987, Columbus, Ohio, US; abstract no.236233, TAKAHASHI HAJIME ET AL.: 'Preparation of high-purity 2,3-difluoro-6-nitrophenol as an intermediate for bactericides.' Seite 741 ;Spalte 1 ; in der Anmeldung erwähnt siehe Zusammenfassung SA 51453 030 & JP,A,62 181 241 (IHARA CHEMICAL INDUSTRY CO., LTD.) 8. August 1987

## Beschreibung

Die vorliegende Erfindung betrifft ein verbessertes Verfahren zur Herstellung von 2,3-Difluor-6-nitrophenol in guten Ausbeuten und hoher Selektivität. Die Verbindung stellt ein wertvolles Vorprodukt zur Herstellung von Chinoloncarbonsäureantibiotika dar.

Die inredestehende Verbindung konnte bisher nur durch Reaktion von Natriumhydroxid mit 2,3,4-Trifluornitrobenzol bei 25 °C, anschließendes Ansäuern bis zum Ausfallen des Produkts und dessen Abfiltrieren gemäß dem Reaktionsschema 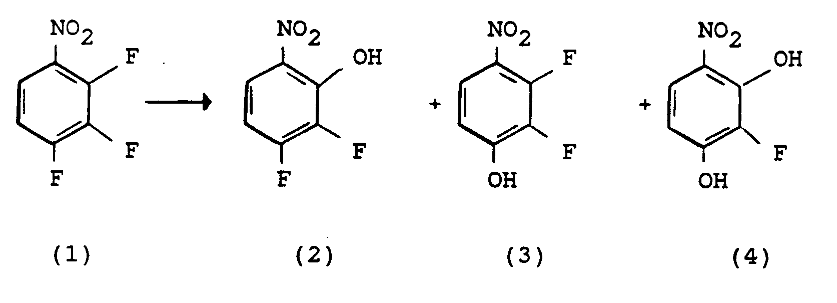
hergestellt werden, wobei Isomerenrestgehalte an 2,3-Difluor-4-nitrophenol (3) auftreten.

Diese Isomerengehalte ließen sich durch Extraktion des Produktes mit Nicht-Halogenkohlenwasserstoffen senken (Ihara Chem. Ind. Co., JP 62/181241 A2), während bei der Extraktion mit Halogenkohlenwasserstoffen nur ein Produkt von ungefähr 90 prozentigem Gehalt anfiel.

Als wesentlich für die Reaktionsführung erweist sich bei dieser Methode wegen der geringen Löslichkeit von 2,3,4-Trifluor-nitrobenzol (1) bzw. der Alkalimetallsalze von (2) in wäßriger Alkalimetallhydroxidlösung der Zusatz von polar aprotischen Lösungsmitteln (Daiichi Seiyaku Co. Ltd., JP 61/246150, 246151, 246171, 246188, JP 58/135840).

Die Abtrennung des zugesetzten Lösungsmittels (in der Regel Dimethylsulfoxid) mußte durch Extraktion mittels eines weiteren organischen Lösungsmittels (Chloroform) aus alkalischer Lösung erfolgen. Nach Ansäuern der abgetrennten wäßrigen Phase erfolgte die Isolierung des Produktes (2) ebenfalls durch Extraktion mit Chloroform (Daiichi Seiyaku Co. Ltd., JP 61/246150, 1.11.86).

Es bestand somit ein Bedürfnis nach einen besseren Verfahren, bei dem der Zusatz von Lösungsvermittlern und die Extraktion mit organischen Lösungsmitteln entfällt.

In Lösung der somit gestellten Aufgabe wurde nun gefunden, daß man isomerenreines 2,3-Difluor-6-nitrophenol in guten Ausbeuten und hoher Selektivität vorteilhaft herstellen kann, indem man 2,3,4-Trifluornitrobenzol mit wäßriger Alkalimetall- oder Erdalkalimetallhydroxidlösung- bzw. -suspension, vorzugsweise Kaliumhydroxidlösung, in Abwesenheit von Lösungsmitteln bei Temperaturen von 20 °C bis 100 °C, vorzugsweise von 30 °C bis 60 °C, umsetzt, das Reaktionsgemisch durch Zugabe von Säure auf einen pH-Wert von 1 bis 6, vorzugsweise von 2,5 bis 4,5, besonders bevorzugt von 1,5 bis 3,0 stellt, das entstandene Produkt durch Einleiten von Wasserdampf in die Reaktionsmischung wasserdampfdestilliert und das 2,3-Difluor-6-nitrophenol nach Abkühlen aus dem Destillat isoliert.

Es ist zweckmäßig, die wäßrige Alkalimetall- oder Erdalkalimetallhydroxidlösung- bzw. -suspension einer vorgelegten Mischung aus Wasser und 2,3,4-Trifluornitrobenzol langsam zuzudosieren. Dadurch wird eine Selektivität von 95 bis 96 % erreicht und die Rührbarkeit der Lösung bleibt erhalten.

Nach dem erfindungsgemäßen Verfahren gelingt die Isolierung des isomerenreinen 2,3-Difluor-6-nitrophenols in 86 %iger Ausbeute der Theorie, wobei auch bei der Isolierung der genannten Verbindung kein organisches Lösungsmittel verwendet wird.

Als wäßrige Alkalimetallhydroxidlösungen können beispielsweise Lithium-, Natrium- oder Kaliumhydroxidlösungen oder Mischungen daraus, vorzugsweise Kaliumhydroxidlösungen, als Erdalkalimetallhydroxidlösungen- bzw. -suspensionen beispielsweise Calcium-, Strontium- oder Bariumhydroxidlösungen- bzw. Suspensionen oder Mischungen daraus, vorzugsweise Calciumhydroxidlösungen- bzw. -suspensionen, angewandt werden. Diese Lösungen werden zweckmäßigerweise in Form von 10 bis 80 prozentigen Lösungen bzw. Suspensionen, vorzugsweise von 30 bis 50 prozentigen Lösungen bzw. Suspensionen eingesetzt.

Das Ansäuern kann mit den üblichen nicht-oxidierenden Mineralsäuren, wie beispielsweise verdünnter Schwefelsäure, Phosphorsäure, Salzsäure, Bromwasserstoffsäure oder Jodwasserstoffsäure, sowie mit ausreichend starken organischen Säuren, wie beispielsweise Ameisensäure oder Essigsäure, erfolgen.

Das so erhaltene Produkt 2,3-Difluor-6-nitrophenol kann nach literaturbekannten Methoden in antibakterielle Mittel überführt werden (Daiichi Seiyaku Co. Ltd., EP 322815 A2; EP 241806 A2; JP 62/246188 A2; EP 206283 A2; JP 57/203085; EP 47005 A1; JP 61/246171; JP 61/246151; JP 61/246172; EP 101829 A2; JP 58/072588 A2; Sankyo Co. Ltd., Ube Ind. Ltd., JP 63/0600990; JP 62/155282; JP 61/204188 A2; Hofmann-LaRoche, F. und Co. A.-G., EP 259804 A2; EP 216345 A2; S. Radl, V. Zikan, Collect. Czech. Chem. Commun., 54(2), 506-15 (1989); K. Sakano, S. Yokohama, I. Hayakawa, S. Atarashi, S. Kadoya, Agric. Biol. Chem., 51(5), 1265-1270 (1987); I. Hayakawa, T. Hiramitsu, Y. Tanaka, Chem. Pharm. Bull., 32(12), 4907-13 (1984)).

Das Verfahren wird zweckmäßigerweise bei Atmosphärendruck durchgeführt; es kann jedoch auch bei Überdruck vorgenommen werden.

Durch die nachstehenden Beispiele wird das erfindungsgemäße Verfahren näher erläutert, ohne darauf beschränkt zu werden.

### Beispiel 1

1,77 kg (10 Mol) 2,3,4-Trifluornitrobenzol werden in 4 l Wasser gegeben. Dann wird die Mischung auf 40 °C erwärmt und kräftig gerührt. Anschließend tropft man 389,4 g (22 Mol) 31,7 prozentige Kaliumhydroxidlösung so zu, daß die Temperatur zwischen 40 und 55 °C bleibt. Nach ca. 4 h ist die Reaktion beendet (GC-Kontrolle). Man bringt das Reaktionsgemisch mit 70 prozentiger Schwefelsäure bei 70 °C auf pH 3. Anschließend wird in die Lösung Wasserdampf eingeleitet, wobei das Produkt mit übergeht und nach Kühlen auf 10 °C isoliert wird. Man erhält nach dem Trocknen 1,51 kg 2,3-Difluor-6-nitrophenol mit einem Gehalt > 99,9 % (GC) (Schmelzpunkt 63,5 °C), was einer Ausbeute von 86 % der Theorie entspricht.

Statt mit der 70 %igen Schwefelsäure kann auch mit beispielsweise 85 %iger Phosphorsäure angesäuert werden.

### Beispiel 2

1,77 kg (10 Mol) 2,3,4-Trifluornitrobenzol werden in 2 l Wasser gegeben, auf 40 °C erwärmt und kräftig gerührt. Anschließend tropft man 1760 g (22 Mol) 31,7 prozentige Natriumhydroxidlösung so zu, daß die Temperatur nicht über 60 °C steigt. Nach ca. 2 h zeigt GC-Kontrolle keine Ausgangsverbindung mehr an. Man bringt das Reaktionsgemisch mit 70 prozentiger Schwefelsäure auf pH 2,5. Anschließend wird in die Lösung Wasserdampf eingeleitet, wobei das Produkt mit dem Wasserdampf übergeht. Die abgeschiedene Lösung wird unter Rühren auf 10 °C gekühlt und der ausgefallene Feststoff abgesaugt. Das Wasser mit einem Restgehalt von ca. 0,1 % 2,3-Difluor-6-nitrophenol kann im Folgeansatz verwendet werden. Man erhält nach dem Trocknen 1,48 kg 2,3-Difluor-6-nitrophenol mit einem Gehalt > 99,9 % (GC) (Schmelzpunkt 64 °C), was einer Ausbeute von 85 % der Theorie entspricht.

Statt mit der 70 %igen Schwefelsäure kann auch mit beispielsweise 36 %iger wässriger Salzsäure angesäuert werden.

### Beispiel 3

1,77 kg (10 Mol) 2,3,4-Trifluornitrobenzol werden in 2,5 l Wasser gegeben, auf 40 °C erwärmt und kräftig gerührt. Anschließend tropft man 1320 g (22 Mol) 40 prozentige Lithiumhydroxidlösung so zu, daß die Temperatur binnen 2,5 h von 40 auf 55 °C steigt. Nach dieser Zeit zeigt GC-Kontrolle keine Ausgangsverbindung mehr an. Man bringt das Reaktionsgemisch mit 70 prozentiger Schwefelsäure auf pH 2. Anschließend wird, wie in den Beispielen 1 und 2 beschrieben, aufgearbeitet. Man erhält nach dem Trocknen 1,48 kg 2,3-Difluor-6-nitrophenol mit einem Gehalt > 99,9 % (GC) (Schmelzpunkt 63,5 °C), was einer Ausbeute von 85 % der Theorie entspricht.

Statt mit der 70 %igen Schwefelsäure kann auch mit Ameisensäure angesäuert werden.

### Beispiel 4

1,77 kg (10 Mol) 2,3,4-Trifluornitrobenzol und 1,56 kg (21 Mol) Calciumhydroxid werden in 5 l Wasser auf 70 °C erhitzt und gut gerührt. Nach etwa 6 h ist die Umsetzung abgeschlossen, wie gaschromatographisch nachgewiesen werden kann. Man säuert mit 70 %iger Schwefelsäure auf pH 1,5 an und arbeitet, wie in den Beispielen 1 und 2 beschrieben, auf (die alkalische Lösung kann vor dem Ansäuern auch bei 60 °C von anorganischen Salzen filtriert werden). Es werden 1,40 kg gelbes 2,3-Difluor-6-nitrophenol isoliert; ein Restgehalt an 2,3-Difluor-4-nitrophenol kann nicht nachgewiesen werden. Die Ausbeute entspricht 80 % der Theorie.

Säuert man mit Bromwasserstoffsäure statt mit Schwefelsäure an, so erhält man im wesentlichen dasselbe Ergebnis. Ebenso analog verlaufen Ansätze unter Verwendung von 2,55 kg (21 Mol) Strontium- oder 3,68 kg (21,5 Mol) Bariumhydroxid anstatt Calciumhydroxid.

## Patentansprüche

1. Verfahren zur Herstellung von isomerenreinem 2,3-Difluor-6-nitrophenol, dadurch gekennzeichnet, daß man 2,3,4-Trifluornitrobenzol mit wäßriger Alkalimetall- oder Erdalkalimetallhydroxidlösung bzw. -suspension in Abwesenheit organischer Lösungsmittel bei Temperaturen von 20 °C bis 100 °C umsetzt, das Reaktionsgemisch durch Zugabe von Säure auf einen pH-Wert von 1 bis 6 stellt, das entstandene Produkt wasserdampfdestilliert und das 2,3-Difluor-6-nitrophenol nach Abkühlen aus dem Destillat isoliert, wobei in keinem Verfahrensschritt ein organisches Lösungsmittel zugegen ist.

2. Verfahren nach Anspruch 1, dadurch gekennzeichnet, daß man bei Temperaturen von 30 °C bis 60 °C umsetzt.

3. Verfahren nach mindestens einem der Ansprüche 1 und 2, dadurch gekennzeichnet, daß man auf einen pH-Wert von 2,5 bis 4,5 stellt.

4. Verfahren nach mindestens einem der Ansprüche 1 bis 3, dadurch gekennzeichnet, daß man auf einen pH-Wert von 1,5 bis 3,0 stellt.

5. Verfahren nach mindestens einem der Ansprüche 1 bis 4, dadurch gekennzeichnet, daß man als wäßrige Alkalimetallhydroxidlösung wäßrige Lithium-, Natrium- oder Kaliumhydroxidlösung oder Mischungen daraus einsetzt.

6. Verfahren nach mindestens einem der Ansprüche 1 bis 4, dadurch gekennzeichnet, daß man als wäßrige Erdalkalimetallhydroxidlösung bzw. -suspension Calcium-, Strontium- oder Bariumhydroxidlösung bzw. -suspension oder Mischungen daraus einsetzt.

7. Verfahren nach mindestens einem der Ansprüche 1 bis 6, dadurch gekennzeichnet, daß man mit einer nichtoxidierenden Mineralsäure ansäuert.

8. Verfahren nach mindestens einem der Ansprüche 1 bis 7, dadurch gekennzeichnet, daß man das Verfahren bei Atmosphären- oder Überdruck durchführt.

## Claims

1. A process for the preparation of isomerically pure 2,3-difluoro-6-nitrophenol, which comprises reacting 2,3,4-trifluoronitrobenzene with agueous alkali metal or alkaline earth metal hydroxide solution or suspension in the absence of organic solvent at temperatures of 20°C to 100°C, adjusting the reaction mixture to a pH of 1 to 6 by addition of acid, steam-distilling the resulting product and isolating the 2,3-difluoro-6-nitrophenol from the distillate after cooling, no organic solvent being present in any process step.

2. The process as claimed in claim 1, wherein the reaction is carried out at temperatures of 30°C to 60°C.

3. The process as claimed in at least one of claims 1 and 2, wherein the mixture is adjusted to a pH of 2.5 to 4.5.

4. The process as claimed in at least one of claims 1 to 3, wherein the mixture is adjusted to a pH of 1.5 to 3.0.

5. The process as claimed in at least one of claims 1 to 4, wherein the aqueous alkali metal hydroxide solution employed is aqueous lithium hydroxide, sodium hydroxide or potassium hydroxide solution or mixtures thereof.

6. The process as claimed in at least one of claims 1 to 4, wherein the aqueous alkaline earth metal hydroxide solution or suspension employed is calcium hydroxide, strontium hydroxide or barium hydroxide solution or suspension or mixtures thereof.

7. The process as claimed in at least one of claims 1 to 6, wherein the acidification is carried out with a non-oxidizing mineral acid.

8. The process as claimed in at least one of claims 1 to 7, wherein the process is carried out at atmospheric pressure or elevated pressure.

## Revendications

1. Procédé pour la préparation de 2,3-difluoro-6-nitrophénol dépourvu d'isomères, caractérisé en ce qu'on fait réagir le 2,3,4-trifluoro-nitrobenzène avec une suspension respectivement solution aqueuse d'hydroxyde de métaux alcalins ou de métaux alcalino-terreux, en l'absence de solvants organiques, à des températures de 20 à 100°C, on établit dans le mélange réactionnel par addition d'acide un pH de 1 à 6, on entraîne à la vapeur le produit formé, puis on isole le 2,3-difluoro-6-nitrophénol du distillat après refroidissement, n'ayant utilisé dans aucune étape du procédé de solvant organique.

2. Procédé selon la revendication 1, caractérisé en ce qu'on effectue la réaction à des températures de 30°C à 60°C.

3. Procédé selon au moins l'une des revendications 1 et 2, caractérisé en ce qu'on établit une valeur de pH de 2,5 à 4,5.

4. Procédé selon au moins l'une des revendications 1 et 3, caractérisé en ce qu'on établit une valeur de pH de 1,5 à 3,0.

5. Procédé selon au moins l'une des revendications 1 et 4, caractérisé en ce qu'on utilise en tant que solution aqueuse d'hydroxyde de métaux alcalins des solutions aqueuses d'hydroxyde de lithium, de sodium ou de potassium, ou leurs mélanges.

6. Procédé selon au moins une des revendications 1 et 4, caractérisé en ce qu'on utilise en tant que suspension, respectivement solution aqueuse d'hydroxyde de métaux alcalino-terreux des suspensions, respectivement solutions d'hydroxyde de calcium, de strontium ou de baryum, ou leurs mélanges.

7. Procédé selon au moins l'une des revendications 1 et 6, caractérisé en ce qu'on acidifie avec un acide minéral non oxydant.

8. Procédé selon au moins l'une des revendications 1 et 7, caractérisé en ce qu'on met en oeuvre le procédé sous une pression atmosphérique ou sous une surpression.
